# EUROPEAN PATENT APPLICATION

(11) **EP 4 431 145 A1**
(43) Date of publication of application: **18.09.2024**
(21) Application number: 22907581.7
(22) Date of filing: 04.04.2022
(51) Int. Cl.: A61M 37/00, B29C 51/10, B29C 33/38, B29C 45/00, B29L 31/00

(54) **METHOD FOR MANUFACTURING MICRONEEDLE**

(30) Priority: 14.12.2021 KR 20210178684
(71) Applicant: Daewoong Therapeutics Inc., Suwon-si, Gyeonggi-do 16226 (KR)
(72) Inventor: KIM, Dong Hwan, Suwon-si, Gyeonggi-do 16336 (KR); KANG, Bokki, Seongnam-si, Gyeonggi-do 13539 (KR); PARK, Sanghan, Seoul 07694 (KR); EUM, Jaehong, Uiwang-si, Gyeonggi-do 16021 (KR); KANG, Yoonsik, Seoul 06013 (KR); IM, Ji Yeon, Daejeon 34308 (KR); LEE, Booyong, Gimpo-si, Gyeonggi-do 10078 (KR)
(74) Representative: RGTH
(86) International application number: PCT/KR2022/004814
(87) International publication number: WO 2023/113108

(57) **Abstract**

An example embodiment of the present disclosure provides a microneedle manufacturing method, including: applying a raw material containing active pharmaceutical ingredients on one or more concave portions formed in a microneedle mold; and pressurizing the raw material at an internal pressure of a pressurizing chamber to fill microstructures of the one or more concave portions with the raw material while loading the microneedle mold, on which the raw material is applied, into the pressurizing chamber, wherein the pressurizing the raw material includes: simultaneously performing an injection and discharge of air into and from the pressurizing chamber to create an air flow inside the pressurizing chamber and to simultaneously perform the filling of the one or more concave portions with the raw material and a drying of the raw material.

## Description

### TECHNICAL FIELD

The present disclosure relates to a microneedle manufacturing method, and more particularly to a microneedle manufacturing method in which pressurizing and drying processes are simultaneously performed on active pharmaceutical ingredients applied on a microneedle mold to simplify a manufacturing process and improve the quantitativeness of the active pharmaceutical ingredients.

### BACKGROUND

In general, a microneedle is used to deliver ingredients via a skin to achieve a desired efficacy. Such a microneedle has a diameter and height of several tens to several thousands of micrometers to pass through a stratum corneum, which is a main barrier layer when delivering the ingredients via the skin. The microneedle enables desired ingredients to reach an epidermal layer or dermis layer, so that the efficacy based on the ingredients may appear at a portion to which the microneedle is applied or throughout the entire body through a circulatory system of the human body.

The microneedle may be broadly classified into a metallic microneedle and a biodegradable microneedle in terms of a material thereof.

The metallic microneedle may be of a type of hollow microneedle, such as a typical injection needle. A path, through which ingredients to be delivered may pass, is formed in the metallic microneedle of this type. The other type of the metallic microneedle is a solid microneedle whose surface is coated with ingredients to be delivered.

The biodegradable microneedle is mainly of the type of solid microneedle. In the biodegradable microneedle, the surface of the biodegradable microneedle is coated with ingredients. Alternatively, the biodegradable microneedle may be formed with a biodegradable polymer. When the biodegradable microneedles are applied to the skin, they undergo decomposition to deliver the coated ingredients to the skin. The biodegradable microneedles are generally manufactured by injecting active pharmaceutical ingredients constituting the biodegradable microneedles into a mold having concave portions formed to correspond to the shape of the biodegradable microneedles.

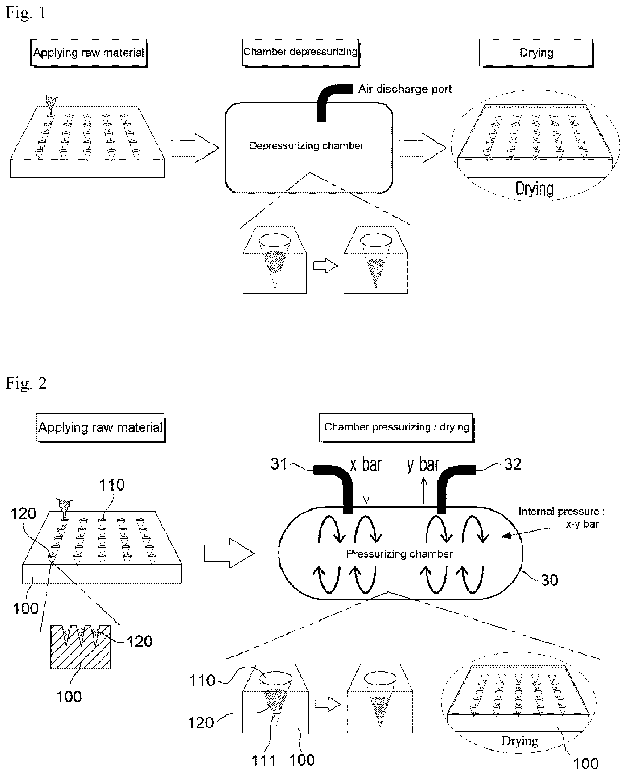

As illustrated in FIG. 1, a biodegradable microneedle manufacturing method in the related art includes applying active pharmaceutical ingredients on the mold, making the active pharmaceutical ingredients permeate into microstructures in the mold in a depressurized or vacuum environment, and drying the active pharmaceutical ingredients in the mold to form the biodegradable microneedles.

However, in the biodegradable microneedle manufacturing method in the related art, the active pharmaceutical ingredients and bubbles in the mold may escape from the mold during a depressurizing or vacuuming process (as the bubbles burst, the active pharmaceutical ingredients may overflow from the concave portions of the mold). This may cause a variation in quantitativeness of the active pharmaceutical ingredients.

### [Document in Related Art]

Korean Registration Patent No. 10-1747099 (filed on June 8, 2017)

### DETAILED DESCRIPTION

The present disclosure was made for the purpose of solving the above matters, and the present disclosure is for the purpose of a microneedle manufacturing method which simultaneously performs pressurizing and drying processes on active pharmaceutical ingredients applied on a microneedle mold to simplify the pressurizing and drying processes and improve quantitativeness of the active pharmaceutical ingredients.

An aspect of the present disclosure to achieve the above purpose provides a microneedle manufacturing method which may include: applying a raw material containing active pharmaceutical ingredients on one or more concave portions formed in a microneedle mold; and pressurizing the raw material at an internal pressure of the pressurizing chamber to fill microstructures of the one or more concave portions with the raw material while loading the microneedle mold, on which the raw material is applied, into a pressurizing chamber. The pressurizing of the raw material includes: simultaneously performing an injection and discharge of air into and from the pressurizing chamber to create an air flow inside the pressurizing chamber and to simultaneously perform the filling of the one or more concave portions with the raw material and a drying of the raw material.

In an aspect, when an injection pressure of the air injected into the pressurizing chamber is X bar and a discharge pressure of the air discharged from the pressurizing chamber is Y bar, the internal pressure of the pressurizing chamber, which is expressed as the X bar minus the Y bar, may be 1.5 bar or more.

In an aspect, the injection pressure of the air injected into the pressurizing chamber may be 2 bar or more, and the discharge pressure of the air discharged from the pressurizing chamber may be 0.5 bar or more.

In an aspect, the microneedle mold may be manufactured by injection-molding a thermoplastic resin.

In an aspect, thermoplastic resin may be at least one selected from the group consisting of polyethyleneterephthalate (PET), polyvinylchloride (PVC), and polypropylene (PP).

In an aspect, the concave portions of the microneedle mold may be sharp-tipped concave portions which are provided independently of each other.

Another aspect of the present disclosure to achieve the above purpose provides a microneedle manufacturing method which may include: placing a plurality of microneedle molds on at least one jig; applying a raw material containing active pharmaceutical ingredients on one or more concave portions formed in each of the plurality of microneedle molds; fixing the at least one jig to a tray; and pressurizing the raw material at an internal pressure of a pressurizing chamber to fill microstructures of the one or more concave portions with the raw material while loading the tray into the pressurizing chamber. The pressurizing of the raw material includes: simultaneously performing an injection and discharge of air into and from the pressurizing chamber to create an air flow inside the pressurizing chamber and to simultaneously perform the filling of the one or more concave portions with the raw material and a drying of the raw material.

In as aspect, when an injection pressure of the air injected into the pressurizing chamber is X bar and a discharge pressure of the air discharged from the pressurizing chamber is Y bar, the internal pressure of the pressurizing chamber, which is expressed as the X bar minus the Y bar, may be 1.5 bar or more.

In an aspect, the injection pressure of the air injected into the pressurizing chamber may be 2 bar or more, and the discharge pressure of the air discharged from the pressurizing chamber may be 0.5 bar or more.

According to an aspect of the present disclosure, a microneedle manufacturing method simultaneously performs pressurizing and drying processes on active pharmaceutical ingredients applied on a microneedle mold, which makes it possible to simplify the pressurizing and drying processes process and improve quantitativeness of the active pharmaceutical ingredients. Further, it is possible to suppress exposure of microneedles to an external environment during manufacture thereof and suppress the microneedles from being contaminated.

Further, by using a jig on which a plurality of microneedle molds may be mounted, and a try capable of fixing a plurality of jigs thereto, it is possible to manufacture a large number of microneedles inside a pressurizing chamber, which improves productivity of the microneedles.

Effects of the present disclosure are not limited to the above-described effects, and other effects may be inferred from the configurations described in the detailed description or the claims of the present disclosure.

### BRIEF DESCRIPTION OF THE DRAWINGS

FIG. 1 is a view conceptually illustrating a microneedle manufacturing method in the related art.
FIG. 2 is a flowchart illustrating a microneedle manufacturing method according to an example embodiment of the present disclosure.
FIG. 3 is a view conceptually illustrating a microneedle manufacturing method in a case where a dot-application manner is applied for concave portions of a mold in an example embodiment of the present disclosure.
FIG. 4 is a view conceptually illustrating a microneedle manufacturing method in a case where an entire-surface application manner is applied for the entire upper surface of the mold in an example embodiment of the present disclosure.
FIG. 5 is a flowchart illustrating a microneedle manufacturing method according to another example embodiment of the present disclosure.
FIG. 6 is a view conceptually illustrating a microneedle manufacturing method in a case where a dot-application manner is applied for concave portions of a mold in another example embodiment of the present disclosure.
FIG. 7 is a view conceptually illustrating a microneedle manufacturing method in a case where an entire-surface application manner is applied for the entire upper surface of the mold in another example embodiment of the present disclosure.
FIGS. 8 to 13 illustrate example embodiments showing whether or not microneedles based on each polymer are successfully produced when the internal pressure of a pressurizing chamber are varied.

### DETAILED DESCRIPTION

The present disclosure will be described below with reference to the accompanying drawings. The present disclosure may be embodied in many different forms and is not limited to the example embodiments described herein. In order to clearly describe the present disclosure, detailed descriptions of parts irrelevant to the present disclosure will be omitted, and the same reference numerals will be given to the same constituent elements throughout the specification.

Throughout the specification, when one constituent element is referred to as being "connected" to another constituent element, the one constituent element may be "directly connected" to another constituent element or may be "indirectly connected" to another constituent element by intervening yet another constituent element therebetween. Further, when one constituent element is referred to as "comprising or including" another constituent element, this means that the one constituent element may further include other constituent elements, rather than excluding other constituent elements, unless stated otherwise.

Example embodiments of the present disclosure will be described in detail below with reference to the accompanying drawings.

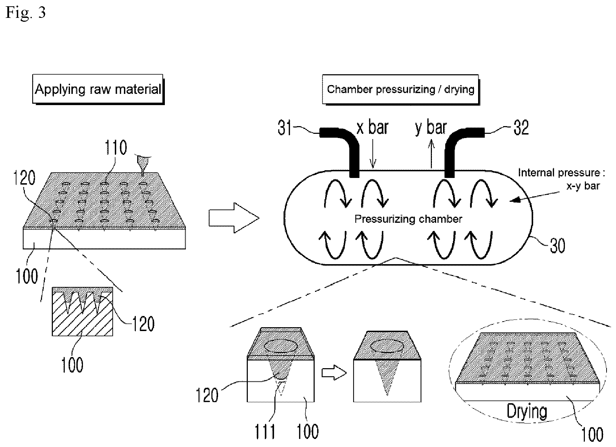

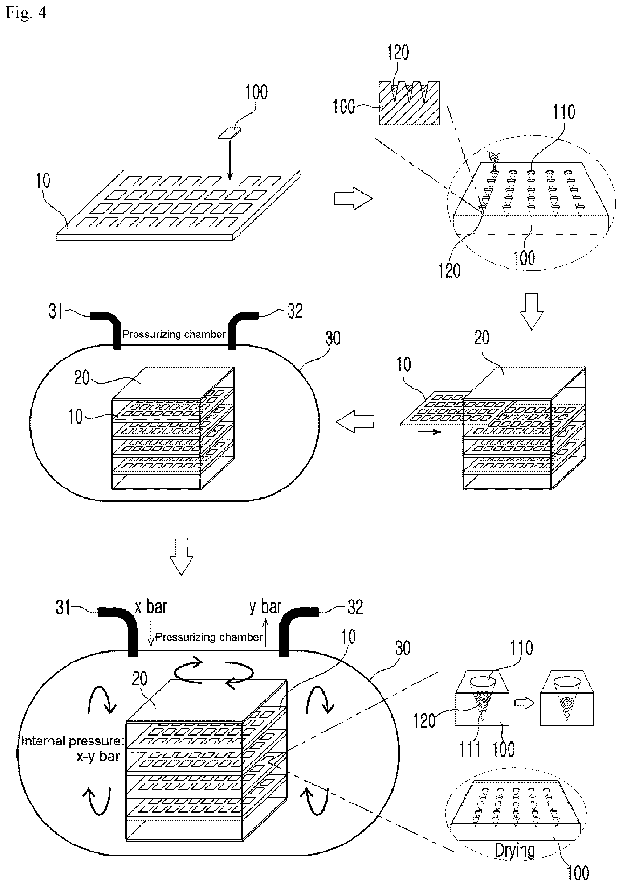

FIG. 2 is a flowchart illustrating a microneedle manufacturing method according to an example embodiment of the present disclosure. FIG. 3 is a view conceptually illustrating a microneedle manufacturing method in a case where a dot-application manner is applied for concave portions of a mold in an example embodiment of the present disclosure. FIG. 4 is a view conceptually illustrating a microneedle manufacturing method in a case where an entire-surface application manner is applied for the entire upper surface of the mold in an example embodiment of the present disclosure.

Referring to FIGS. 2 to 4, the microneedle manufacturing method of the present disclosure includes Operation S 110 of preparing a microneedle mold 100, Operation S120 of applying a raw material 120 on concave portions 110 of the microneedle mold 100, and Operation S130 of pressurizing the raw material inside a pressurizing chamber 30 to perform filling concave portions with the raw material and drying the raw material.

More specifically, the microneedle manufacturing method of the present disclosure includes Operation S110 of preparing the microneedle mold 100, Operation S120 of applying the raw material 120 containing active pharmaceutical ingredients on one or more concave portions 110 formed in the microneedle mold 100, and Operation S130 of pressurizing the raw material 120 at an internal pressure of the pressurizing chamber 30 to fill microstructures 111 in the one or more concave portions 110 with the raw material 120 while loading the microneedle mold 100 having the raw material 120 applied thereon into the pressurizing chamber 30. Operation S130 of pressurizing the raw material 120 includes simultaneously performing injection and discharge of air into and from the pressurizing chamber 30 to create an air flow inside the pressurizing chamber 30 and to simultaneously perform the filling of concave portions 110 with the raw material 120 and the drying of the raw material 120.

In Operation S110 of preparing the microneedle mold 100, an injection device may be used to injection-mold a thermoplastic resin through a sequence of melting, flowing, cooling, and removing processes, to manufacture the microneedle mold 100 having the one or more concave portions 110. In this case, one surface of the microneedle mold 100 may have the one or more concave portions 110, and the other surface thereof may be flat. The concave portions 110 may serve as molds for microneedles to be formed in a subsequent operation, which have shapes corresponding to the microneedles.

Each concave portion 110 may have a sharp-tipped concave portion. As used herein, the term "sharp-tipped concave portion" means a concave portion depressed downward from a flat upper surface such that a cross-sectional area thereof is reduced from the flat upper surface toward a lower surface forming a tip, like a shape of a typical microneedle. The term "upper surface" and/or "lower surface" used herein is for specifying a relative positional relationship in each configuration and does not specify absolute positions thereof.

Further, the microneedle mold 100 may be made of a solid material. The microneedle mold 100 may be made of any suitable material, preferably a plastic resin.

More specifically, the microneedle mold 100 may be made of at least one or more selected from the group consisting of polyethyleneterephthalate (PET), polyvinylchloride (PVC), and polypropylene (PP), but is not limited thereto.

A microneedle made of a silicon material in the related art is expensive, thus the silicon microneedle in the related art often needs to be reused. In the reusing process of the silicon microneedle, verification of a cleaning validation (CV) of fine concave portions in which microneedles are molded may be a difficult process to conduct. Verification of a permeability of a cleaning solvent to silicon may also be a difficult process. Further, the silicon material is physicochemically decomposable and has a relatively weak hardness. As a result, fragments may be generated during a manufacturing or cleaning process. In addition, it is difficult to determine a lifespan of a mold made of the silicon material.

In addition, in the case in which the mold is manufactured using the silicon material, the silicon does not play a protective role against ambient air or external foreign matters. As a result, dried microneedles need to be removed from the mold to repackage the same. In this case, since it is practically difficult to individually pack each of the fine-sized microneedles in an airtight manner, the microneedles may be packaged in a pouch or a container while being exposed to ambient air.

The sealability and stability of a packaging container have a significant effect on maintaining the quality of pharmaceuticals in the packaging container. Therefore, the packaging container needs to completely prevent physicochemical deformation or deterioration of the pharmaceuticals in the packaging container through a transport condition simulation and mechanical test relating to final products.

The microneedle mold 100 of the present disclosure may function as a primary packaging container completely contacting with the microneedles. The microneedles molded in the concave portions 100 does not need to be removed and repackaged. The microneedle mold 100 may be configured to be removed before a final use. This makes it possible to simplify the process of packaging the microneedles and easily maintain the quality of the microneedles.

In Operation S120 of applying the raw material 120 on the concave portions 110 of the microneedle mold 100, the raw material 120 may be supplied from a reservoir to the microneedle mold 100 via a single discharge port so that the raw material 120 is applied on each concave portion 110. However, a method of applying the raw material 120 is not limited to the above, but the raw material 120 may be applied on the concave portions 110 via a plurality of discharge ports arranged respectively adjacent to the plurality of concave portions 110 as needed.

In an example embodiment, as illustrated in FIGS. 3 and 4, the application of the raw material 120 on the concave portions 110 may be performed in a dot-application manner in which one or more droplets are applied on each of the concave portions 110, or in an entire-surface application manner in which the raw material 120 is applied on the entire upper surface of the microneedle mold 100 having the concave portions 110 formed therein.

The raw material 120 may include one or more selected from the group consisting of a metal, a non-metal, a biodegradable polymer, and active pharmaceutical ingredients depending on an intended use of the microneedles. For example, the raw material 120 may be a composition containing the active pharmaceutical ingredients, or may be a composition containing the metal, the non-metal, the biodegradable polymer, or a combination of two or more thereof, but is not limited thereto.

In Operation S130 of pressurizing the raw material 120 applied on the concave portions 110 in the pressurizing chamber 30, the raw material 120 may be pressurized by the internal pressure of the pressurizing chamber 30 to fill the fine structures 111 of the concave portions 110 with the raw material 120.

In a state in which the pressurizing chamber 30 is sealed, air is injected into the pressurizing chamber 30 via an injection port 31 so that the internal pressure of the pressurizing chamber 30 is increased. At this time, the raw material 120 is pressurized by the internal pressure of the pressurizing chamber 30 so that fine bubbles contained in the raw material 120 are separated and removed from the raw material 120. As a result, the raw material 120 is filled into the fine structures 111 of the concave portions 110.

At the same time, the air is discharged from the pressurizing chamber 30 via a discharge port 32 so that an air flow is created inside the pressurizing chamber 30 to simultaneously perform the drying of the raw material 120.

That is, the microneedle manufacturing method of the present disclosure may simultaneously perform pressurizing and dry processes on the raw material 120 applied on the concave portions 110 of the microneedle mold 100 inside the pressurizing chamber 30. This simplifies the processes, improves quantitativeness of the raw material 120, and reduces exposure to the external environment during the manufacturing process, which makes it possible to suppress the microneedles from being contaminated.

Further, when an injection pressure of the air injected into the pressurizing chamber 30 via the injection port 31 is X bar and a discharge pressure of the air discharged from the pressurizing chamber 30 via the discharge port 32 is Y bar, the internal pressure of the pressurizing chamber 30, which is expressed by the X bar minus the Y bar, may be 1.5 bar or more, preferably 2 bar or more.

In an example embodiment, the injection pressure of the air injected into the pressurizing chamber 30 may be 2 bar or more, preferably 2.5 bar or more, and the discharge pressure of the air discharged from the pressurizing chamber 30 may be 0.5 bar or more.

That is, both the injection and discharge of the air may be simultaneously performed in the pressurizing chamber 30. The filling of the concave portions with the raw material and the drying of the raw material 120 may be successfully performed when the internal pressure of the pressurizing chamber 30 is maintained at 1.5 bar or more, preferably 2 bar or more. When the internal pressure of the pressurizing chamber 30 is less than 1.5 bar, the fine structures 111 of the concave portions 110 may not be completely filled with the raw material 120 and the fine bubbles contained in the raw material 120 may not be separated from the raw material 120.

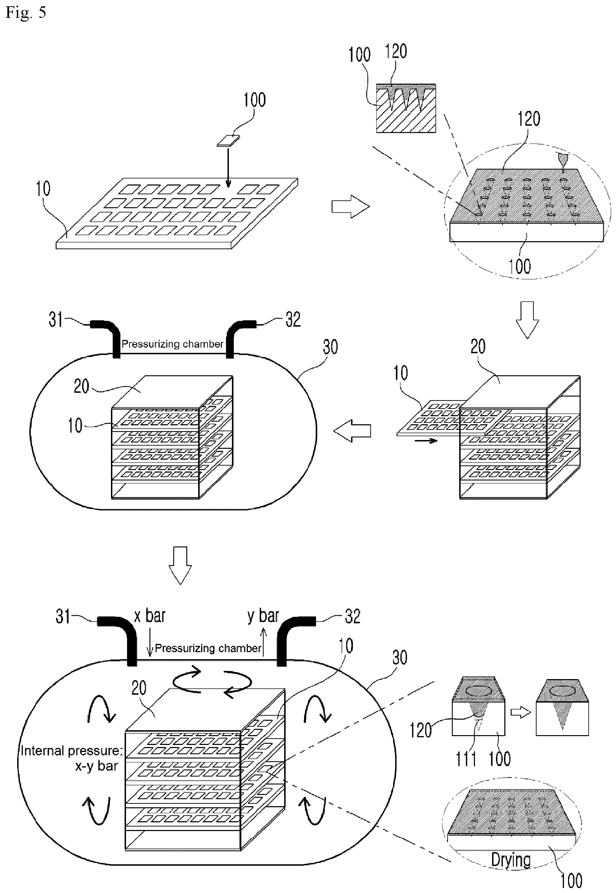

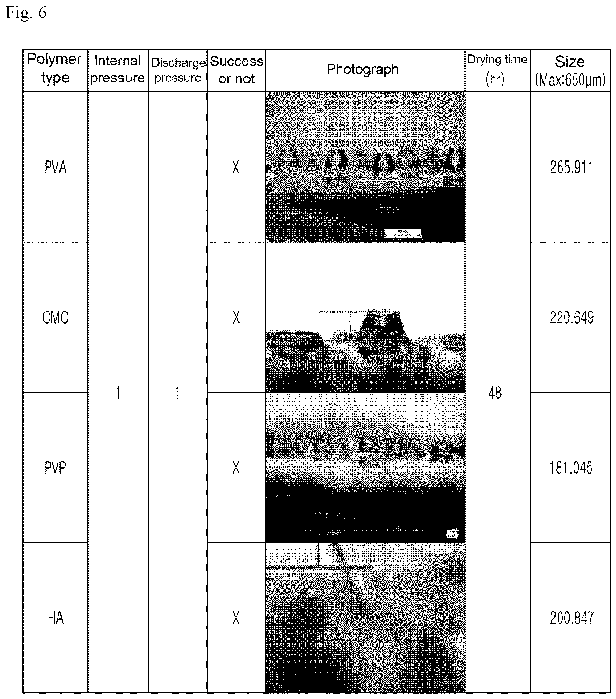

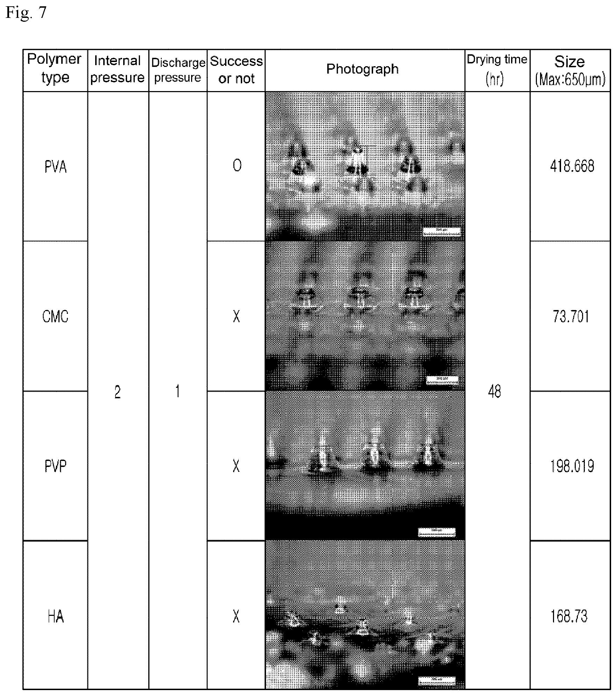

FIG. 5 is a flowchart illustrating a microneedle manufacturing method according to another example embodiment of the present disclosure. FIG. 6 is a view conceptually illustrating a microneedle manufacturing method in a case where a dot-application manner is applied for concave portions of a mold in another example embodiment of the present disclosure. FIG. 7 is a view conceptually illustrating a microneedle manufacturing method in a case where an entire-surface application manner is applied for the entire upper surface of the mold in another example embodiment of the present disclosure.

Hereinafter, differences from the above-described example embodiment or additional features of another example embodiment will be mainly described.

Referring to FIGS. 5 to 7, the microneedle manufacturing method of this present disclosure includes Operation S210 of preparing the microneedle mold 100, Operation S220 of placing a plurality of microneedle molds 100 on each of one or more jigs 10, Operation S230 of applying the raw material 120 on the concave portions 110 of each of the microneedle molds 100, Operation S240 of fixing the one or more jigs 10 to a tray 20, and Operation S250 of pressurizing the raw material 120 at an internal pressure of the pressurizing chamber 30 to fill microstructures of the concave portions 110 with the raw material 120 while loading the tray 20 into the pressurizing chamber 30. Operation S250 of pressurizing the raw material 120 includes: simultaneously performing injection and discharge of air into and from the pressurizing chamber 30 to create an air flow inside the pressurizing chamber 30 and to simultaneously perform a filling of the concave portions 110 with the raw material 120 and a drying of the raw material 120.

In Operation S220 of placing the plurality of microneedle molds 100 on the one or more jigs 10, each jig 10 may be formed in a plate shape and may have an upper surface on which a plurality of mounting portions (not shown) are provided. The plurality of microneedle molds 100 may be positioned at the plurality of mounting portions, respectively. Each mounting portion may have a groove structure of a shape corresponding to that of each microneedle mold 100.

The jig 10 may be configured such that the microneedle molds 100 are mounted in the form of N×M (where N and M are numeric characters). A total of N×M microneedle molds 100 may be mounted on one jig 10.

In Operation S230 of applying the raw material 120 on the concave portions 110 of each microneedle mold 100, the raw material 120 may be supplied from a reservoir to the microneedle mold 100 via a single discharge port so that the raw material 120 is applied on each concave portion 110. However, a method of applying the raw material 120 is not limited to the above, but the raw material 120 may be applied on the concave portions 110 via a plurality of discharge ports arranged respectively adjacent to the plurality of concave portions 110 as needed.

In this case, as illustrated in FIGS. 6 and 7, the application of the raw material 120 on the concave portions 110 may be performed in the dot-application manner in which one or more droplets are applied on each of the concave portions 110, or in the entire-surface application manner in which the raw material 120 is applied on the entire upper surface of the microneedle mold 100 having the concave portions 110 formed therein.

In Operation S240 of fixing the one or more jigs 10 to the tray 20, the tray 20 may be a multi-stage tray. A plurality of jigs 10 may be fixed to one tray 20 in multiple stages. Further, the plurality of jigs 10 may be fixed to one stage of the tray 20.

As described above, by using the jig 10 on which the plurality of microneedle molds 100 may be mounted, and the try 20 capable of fixing the plurality of jigs 10 thereto, it is possible to manufacture a large number of microneedles inside the pressurizing chamber 30, which improves productivity of the microneedles.

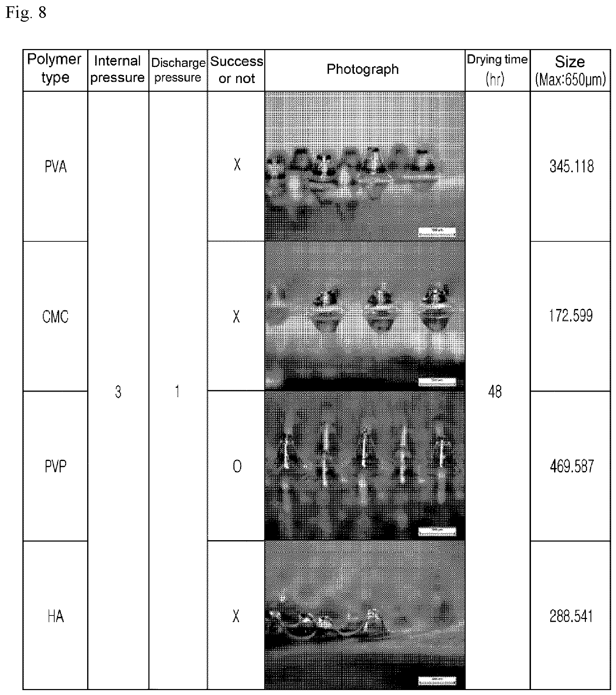

FIGS. 8 to 13 illustrate example embodiments showing whether or not microneedles based on each polymer are successfully produced when the internal pressure of a pressurizing chamber are varied.

Referring to FIGS. 8 to 13, four types of polymers such as polyvinylalcohol (PVA), carboxymethylcellulose (CMC), polyvinylpyrrolidone (PVP), and hyaluronic acid (HA) were used as the raw material, and whether or not the microneedles based on each polymer were successfully produced was determined after the pressurizing and drying processes inside the pressurizing chamber. In this case, the maximum (Max) size of the ideal microneedle was 650 micrometer (µm). Whether or not the microneedles were successfully produced was determined depending on whether or not tip portions of the microneedles were formed and the tip portions have a size of 400 µm or more.

In FIG. 8, in an environment in which the injection pressure of the air injected into the pressurizing chamber is 2 bar, the discharge pressure of the air discharged from the pressurizing chamber is 1 bar, and the internal pressure of the pressurizing chamber is 1 bar with respect to four types of polymers applied on each microneedle mold, the pressurizing and drying processes were performed for 48 hours. In this case, it was confirmed that for four types of polymers, the tip portions of the microneedles were produced, and the microneedles having the size of 400 meters (m) or more were not produced.

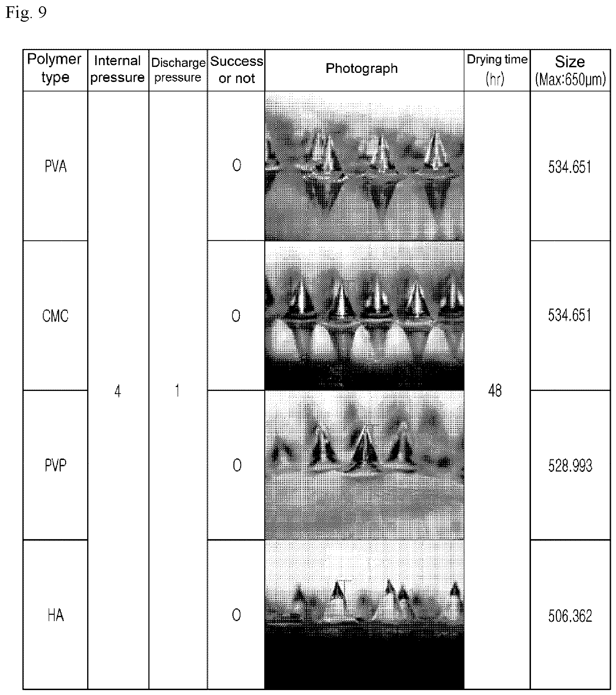

Referring to FIG. 9, in an environment in which the injection pressure of the air injected into the pressurizing chamber is 3 bar, the discharge pressure of the air discharged from the pressurizing chamber is 1 bar, and the internal pressure of the pressurizing chamber is 2 bar with respect to four types of polymers applied on each microneedle mold, the pressurizing and drying processes were performed for 48 hours. In this case, it was confirmed that for the polymer of polyvinylalcohol (PVA), the tip portions of the microneedles were produced, and the microneedles having the size of 400 µm or more were produced, and it was confirmed that for the remaining three types of polymers, the microneedles were not produced.

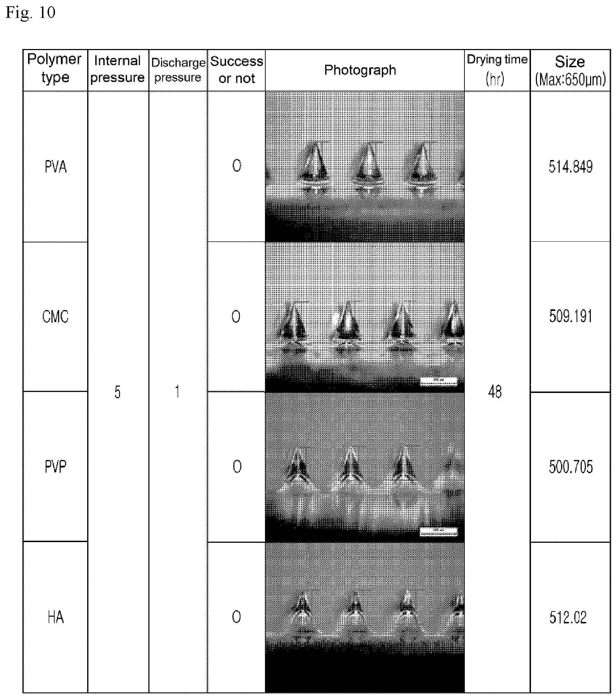

Referring to FIG. 10, in an environment in which the injection pressure of the air injected into the pressurizing chamber is 4 bar, the discharge pressure of the air discharged from the pressurizing chamber is 1 bar, and the internal pressure of the pressurizing chamber is 3 bar with respect to four types of polymers applied on each microneedle mold, the pressurizing and drying processes were performed for 48 hours. In this case, it was confirmed that for the polymer of polyvinylpyrrolidone (PVP), the tip portions of the microneedles were produced, and the microneedles having the size of 400 µm or more were produced, but it was confirmed that for the remaining three types of polymers, the microneedles were not produced.

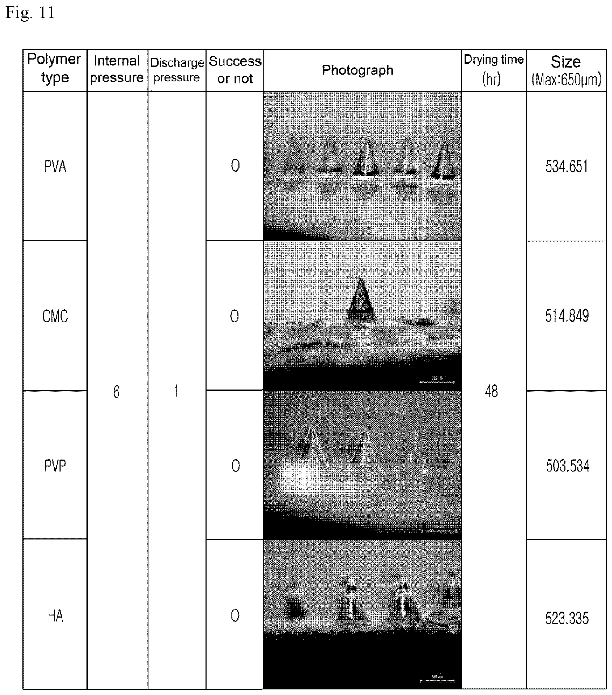

Referring to FIG. 11, in an environment in which the injection pressure of the air injected into the pressurizing chamber is 5 bar, the discharge pressure of the air discharged from the pressurizing chamber is 1 bar, and the internal pressure of the pressurizing chamber is 4 bar with respect to four types of polymers applied on each microneedle mold, the pressurizing and drying processes were performed for 48 hours. In this case, it was confirmed that for all the four types of polymers, the tip portions of the microneedles were produced, and the microneedles having the size of 400 µm or more were produced.

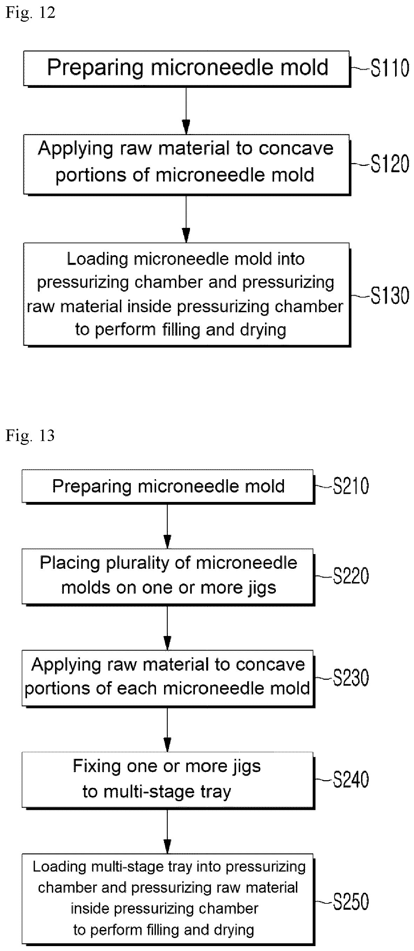

Referring to FIG. 12, in an environment in which the injection pressure of the air injected into the pressurizing chamber is 6 bar, the discharge pressure of the air discharged from the pressurizing chamber is 1 bar, and the internal pressure of the pressurizing chamber is 5 bar with respect to four types of polymers applied on each microneedle mold, the pressurizing and drying processes were performed for 48 hours. In this case, it was confirmed that for all the four types of polymers, the tip portions of the microneedles were produced, and the microneedles having the size of 400 µm or more were produced.

Referring to FIG. 13, in an environment in which the injection pressure of the air injected into the pressurizing chamber is 7 bar, the discharge pressure of the air discharged from the pressurizing chamber is 1 bar, and the internal pressure of the pressurizing chamber is 6 bar with respect to four types of polymers applied on each microneedle mold, the pressurizing and drying processes were performed for 48 hours. In this case, it was confirmed that for all the four types of polymers, the tip portions of the microneedles were produced, and the microneedles having the size of 400 µm or more were produced.

The foregoing description of the present disclosure is merely an example. It will be understood by those skilled in the art that variations are readily possible in other specific forms without changing the technical ideas or essential features of the present disclosure. Therefore, it should be noted that the example embodiments described above are exemplary in all respects and are not restrictive. For example, each constituent element that is described in a single form may be embodied in a distributed fashion. Similarly, constituent elements that are described in the distributed fashion may be embodied in a combined fashion.

The scope of the present disclosure should be construed within the appended claims rather than the foregoing detailed description, and it is intended that all changes or modifications that come from the meaning and scope of the claims and their equivalent concept be embraced therein.

### EXPLANATION OF REFERENCE NUMERALS

100: Microneedle mold
110: Concave portion
111: Microstructure
120: Raw material
10: Jig
20: Tray
30: Pressurizing chamber
31: Injection port
32: Discharge port

## Claims

1. A microneedle manufacturing method, comprising:
applying a raw material containing active pharmaceutical ingredients on one or more concave portions formed in a microneedle mold; and
pressurizing the raw material at an internal pressure of the pressurizing chamber to fill microstructures of the one or more concave portions with the raw material while loading the microneedle mold, on which the raw material is applied, into a pressurizing chamber,
wherein the pressurizing of the raw material includes: simultaneously performing injection and discharge of air into and from the pressurizing chamber to create an air flow inside the pressurizing chamber and to simultaneously perform a filling of the one or more concave portions with the raw material and a drying of the raw material.

2. The microneedle manufacturing method of Claim 1, wherein, when an injection pressure of the air injected into the pressurizing chamber is X bar and a discharge pressure of the air discharged from the pressurizing chamber is Y bar, the internal pressure of the pressurizing chamber, which is expressed as the X bar minus the Y bar, is 1.5 bar or more.

3. The microneedle manufacturing method of Claim 2, wherein the injection pressure of the air injected into the pressurizing chamber is 2 bar or more, and the discharge pressure of the air discharged from the pressurizing chamber is 0.5 bar or more.

4. The microneedle manufacturing method of Claim 1, wherein the microneedle mold is manufactured by injection-molding a thermoplastic resin.

5. The microneedle manufacturing method of Claim 4, wherein the thermoplastic resin is at least one selected from the group consisting of polyethyleneterephthalate (PET), polyvinylchloride (PVC), and polypropylene (PP).

6. The microneedle manufacturing method of Claim 1, wherein the concave portions of the microneedle mold are sharp-tipped concave portions which are provided independently of each other.

7. A microneedle manufacturing method, comprising:
placing a plurality of microneedle molds on at least one jig;
applying a raw material containing active pharmaceutical ingredients on one or more concave portions formed in each of the plurality of microneedle molds;
fixing the at least one jig to a tray; and
pressurizing the raw material at an internal pressure of a pressurizing chamber to fill microstructures of the one or more concave portions with the raw material while loading the tray into the pressurizing chamber,
wherein the pressurizing of the raw material includes: simultaneously performing an injection and discharge of air into and from the pressurizing chamber to create an air flow inside the pressurizing chamber and to simultaneously perform the filling of the microstructures with the raw material and a drying of the raw material.

8. The microneedle manufacturing method of Claim 7, wherein, when an injection pressure of the air injected into the pressurizing chamber is X bar and a discharge pressure of the air discharged from the pressurizing chamber is Y bar, the internal pressure of the pressurizing chamber, which is expressed as the X bar minus the Y bar, is 1.5 bar or more.

9. The microneedle manufacturing method of Claim 8, wherein the injection pressure of the air injected into the pressurizing chamber is 2 bar or more, and the discharge pressure of the air discharged from the pressurizing chamber is 0.5 bar or more.
